# EUROPEAN PATENT APPLICATION

(11) **EP 3 636 247 A1**
(43) Date of publication of application: **15.04.2020**
(21) Application number: 18814466.1
(22) Date of filing: 28.02.2018
(51) Int. Cl.: A61K 8/44, A61K 8/19, A61K 8/25, A61K 8/29, A61Q 1/00

(54) **POWDER MODIFYING AGENT, COMPOSITE POWDER AND MAKEUP COSMETIC**

(30) Priority: 09.06.2017 JP 2017114444
(71) Applicant: Ajinomoto Co., Inc., Tokyo 104-8315 (JP)
(72) Inventor: SUZUKI, Takahiro, Kawasaki-shi Kanagawa 210-8681 (JP)
(74) Representative: Mewburn Ellis LLP
(86) International application number: PCT/JP2018/007454
(87) International publication number: WO 2018/225310

(57) **Abstract**

The present invention relates to a powder modifying agent containing N^{ε}-octanoyl-L-lysine, a composite powder modified using same, and a makeup cosmetic containing the composite powder.

According to the present invention, a powder modifying agent that can impart a powder for cosmetics with the properties appropriate as a powder for makeup cosmetics, namely, superior dispersibility and superior caking property when blended with makeup cosmetics, superior texture (e.g., smoothness, moist feeling etc.) when applied to the skin, and water-repellency, adhesiveness and transparency, can be provided, and further, a composite powder capable of exhibiting the above-mentioned properties in good balance, and a makeup cosmetic superior in sense of use, water-repellency, adhesiveness and transparency can be provided.

## Description

### [Technical Field]

The present invention relates to a powder modifying agent containing N^{ε}-octanoyl-L-lysine, a composite powder obtained using same, and a makeup cosmetic containing the composite powder.

### [Background Art]

As cosmetics containing a powder for cosmetics as a main component, makeup cosmetics such as foundation, face powder, pressed powder, cheek color, eyeliner, eyebrow and the like are commercially available. The powder for cosmetics contained in these cosmetics is required to have various properties. For example, it is required to have dispersibility when liquid or paste form makeup cosmetics are produced, it is required to have caking property when a solid cosmetic form is produced by pressing, it is required to produce a superior texture (e.g., smoothness, moist feeling) when applied to the skin, and it is required to be superior in water-repellency, adhesiveness and transparency.

It is often difficult to satisfy the above-mentioned properties with only the properties that the powder itself has and various powder modifying agents are proposed (e.g., patent document 1). However, these properties are frequently in the trade-off relationship and a powder modifying agent that can impart well-balanced properties has been desired.

### [Document List]

### [Patent documents]

patent document 1: JP-A-2007-238497

### [SUMMARY OF THE INVENTION]

### [Problems to be Solved by the Invention]

Thus, the problem to be solved by the present invention is to provide a powder for cosmetics which affords a makeup cosmetic that is superior in water-repellency, adhesiveness and transparency, shows superior dispersibility and caking property when producing a makeup cosmetic and achieves superior texture (e.g., smoothness, moist feeling) when applied to the skin. Specifically, it is to provide a powder modifying agent that can impart the aforementioned properties, a composite powder using same, and a makeup cosmetic containing the composite powder.

### [Means of Solving the Problems]

The present inventors have found that the above-mentioned properties required when producing a makeup cosmetic can be exhibited in good balance by using a powder modifying agent containing N^{ε}-octanoyl-L-lysine, preparing a composite powder using same, and producing a makeup cosmetic containing the composite powder, which resulted in the completion of the present invention.

That is, the present invention provides the following.
[1] A powder modifying agent comprising N^{ε}-octanoyl-L-lysine.
[2] A composite powder comprising a powder to be a core and N^{ε}-octanoyl-L-lysine.
[3] The composite powder of [2] comprising 0.1 mass % - 50 mass % of the N^{ε}-octanoyl-L-lysine.
[4] The composite powder of [2] or [3] having an average particle size of 0.001 µm - 400 µm and a specific surface area of 1 m²/g - 700 m²/g.
[5] The composite powder of any of [2] to [4] wherein the powder to be the core is in the form of a plate.
[6] The composite powder of any of [2] to [5] wherein the powder to be the core is one or more kinds selected from inorganic powders.
[7] The composite powder of any of [2] to [6] wherein the powder to be the core is one or more kinds selected from titanium oxide, zinc oxide, talc, mica, fluorphlogopite and silica.
[8] The composite powder of any of [2] to [7] further comprising N^{ε}-lauroyl-L-lysine.
[9] A method for producing the composite powder of any of [2] to [7] comprising modifying the powder to be the core by a surface treatment with N^{ε}-octanoyl-L-lysine in water.
[10] The production method of [8] wherein the modification of the powder to be the core is performed by a stepwise surface treatment with N^{ε}-octanoyl-L-lysine and N^{ε}-lauroyl-L-lysine in water.
[11] The production method of [8] wherein the modification of the powder to be the core is performed by a simultaneously surface treatment with N^{ε}-octanoyl-L-lysine and N^{ε}-lauroyl-L-lysine in water.
[12] The composite powder of any of [2] to [8] wherein the composite powder is used as a pigment for cosmetics.
[13] The makeup cosmetic comprising the composite powder of any of [2] to [8].

### [Effect of the Invention]

According to the present invention, a powder modifying agent that can impart a powder for cosmetics with the properties required as a powder for makeup cosmetics can be provided.

That is, the powder modifying agent of the present invention can impart a powder for cosmetics with superior dispersibility and superior caking property when blended with makeup cosmetics, and can impart superior texture (e.g., smoothness, moist feeling etc.) when applied to the skin, and water-repellency, adhesiveness and transparency.

A composite powder prepared using the powder modifying agent of the present invention can exhibit the above-mentioned properties in good balance when blended with makeup cosmetics.

Therefore, makeup cosmetics containing the composite powder of the present invention are superior in the texture when applied to the skin, and superior in water-repellency, adhesiveness and transparency.

### <powder modifying agent>

The powder modifying agent of the present invention (hereinafter sometimes referred to as "the modifying agent of the present invention") contains N^{ε}-octanoyl-L-lysine.

N^{ε}-octanoyl-L-lysine contained in the modifying agent of the present invention can be prepared by a known production method such as a dehydration-condensation reaction of fatty acid and L-lysine and used. It can be commercially obtained from Ajinomoto Co., Inc. under a trade name "Amihope OL".

N^{ε}-octanoyl-L-lysine can be used in free or salt form.

Examples of the salt include alkali metal salts such as sodium salt, potassium salt and the like; alkaline earth metal salts such as magnesium salt, calcium salt and the like; inorganic acid salts such as hydrochloride, nitrate, sulfate, carbonate and the like; organic acid salts such as acetate, lactate, citrate and the like; amino acid salts such as glutamate, aspartate and the like, and the like.

A free form is most preferably used for the object of the present invention.

The modifying agent of the present invention can contain other modifying agents as long as the characteristics of the present invention are not impaired.

Particularly preferable examples of other modifying agent include N^{ε}-lauroyl-L-lysine.

A method for modifying a powder with the modifying agent of the present invention is not particularly limited, and a dry method, a wet method, an integral blend method and the like can be mentioned.

Examples of the dry method include a method containing directly adding the modifying agent of the present invention to a powder to be modified, and copulverizing the mixture using a grinding machine such as a ball mill, an atomizer, a colloid mill and the like, a method including charging a powder to be modified in a rotary mixer, adding dropwise or spraying an acidic solution or basic solution of the modifying agent of the present invention while stirring, further stirring the mixture, and classifying the mixture by sieving and the like.

Examples of the dry method include a method containing preparing a slurry of a powder to be modified and an organic solvent, adding the modifying agent of the present invention to the slurry while stirring, mixing and stirring the mixture, and then filtering, drying and classifying same by sieving, a method containing adding the powder to be modified to an acidic or basic solution of the modifying agent of the present invention and stirring the mixture and causing a precipitation reaction by changing the pH, and other methods.

The integral blend method is a modification method including simultaneously adding and mixing a modifying agent and a powder to be modified. When a powder to be the core and a modifying agent are mixed, the modifying agent is directly added as it is or added after diluting with alcohol or the like into a blending machine and stirred therein.

As a method for modifying a powder with the modifying agent of the present invention, a method including adding a powder to be the core to a solution obtained by dissolving N^{ε}-octanoyl-L-lysine in an alkaline aqueous solution, mixing them, neutralizing the mixture, precipitating N^{ε}-octanoyl-L-lysine on the powder to be the core, performing a surface treatment, and achieving modification can be preferably employed.

When the modifying agent of the present invention further contains N^{ε}-lauroyl-L-lysine, the powder to be the core may be modified by a stepwise surface treatment using solutions obtained by separately dissolving N^{ε}-octanoyl-L-lysine and N^{ε}-lauroyl-L-lysine in alkaline solutions. It may also be modified with N^{ε}-octanoyl-L-lysine and N^{ε}-lauroyl-L-lysine simultaneously by a surface treatment including adding the powder to be the core to a solution in which both N^{ε}-octanoyl-L-lysine and N^{ε}-lauroyl-L-lysine are dissolved and mixing them.

### <composite powder>

The present invention provides a composite powder (hereinafter to be also referred to as "the composite powder of the present invention") by modifying the powder using the powder modifying agent of the present invention.

The composite powder of the present invention contains a powder to be modified, that is, a powder to be the core and N^{ε}-octanoyl-L-lysine.

While the form of the composite powder of the present invention is not particularly limited, the powder to be the core and the modifying agent of the present invention need to be brought into contact with each other.

As the powder to be the core, any powder can be used without a particular limitation as long as it is used for cosmetics.

Examples of an inorganic powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, lepidolite, silica (silicic acid, silicic anhydride etc.), aluminum silicate, magnesium silicate, aluminum silicate magnesium, calcium silicate, barium silicate, strontium silicate, tungsten acid metal salt, hydroxyapatite, vermiculite, gibbsite (e.g., hidirite (registered trade mark) etc.), bentonite, montmorillonite, hectorite, zeolite, ceramic powder, calcium monohydrogen phosphate (calcium secondary phosphate), alumina, aluminum hydroxide, boron nitride, boron nitride and the like.

Examples of an organic powder include polyamide resin powder (e.g., nylon 6 powder, nylon 12 powder etc.), polyester powder, polyethylene powder, polypropylene powder, silicon resin powder (e.g., silicone resin powder etc.), silicone elastomer powder, vinyl resin powder (e.g., polystyrene powder, divinylbenzene-styrene copolymer powder etc.), polyurethane powder, benzoguanamine powder, polymethylbenzoguanamine powder, fluorine resin powder (e.g., polytetrafluoroethylene powder etc.), cellulose powder, silk powder, acrylic resin powder (e.g., polymethylmethacrylate powder, styrene/acrylic acid copolymer powder etc.), acrylic elastomer powder, urea resin powder, phenol resin powder, melamine resin powder, epoxy resin powder, polycarbonate resin powder, microcrystalline fiber powder, starch powder, N-lauroyl lysine, surfactant metal salt powder(metal soap) (e.g., zinc stearate, aluminum stearate, calcium stearate, magnesium stearate, zinc myristate, magnesium myristate, zinc cetyl phosphate, calcium cetyl phosphate, sodium zinc cetyl phosphate etc.).

Examples of a colored pigment include inorganic red pigments such as iron oxide, iron hydroxide, iron titanate and the like; inorganic brown pigments such as γ-iron oxide and the like; inorganic yellow pigments such as yellow iron oxide, yellow ocher and the like; inorganic black pigments such as black iron oxide, carbon black and the like; inorganic violet pigments such as manganese violet, cobalt violet and the like; inorganic green pigments such as chrome hydroxide, chrome oxide, cobalt oxide, cobalt titanate and the like; inorganic blue pigments such as iron blue, ultramarine blue and the like; lakes made from tar dye (pigment) (Red No. 202, Red No. 204, Red No. 205, Red No. 220, Red No. 228, yellow No. 401, Blue No. 404, orange No. 203, orange No. 204 etc.), tar dye (dye) (Red No. 3, Red No. 104, Red No. 106, Red No. 201, Red No. 226, Red No. 227, Red No. 230, Red No. 401, Red No. 505, yellow No. 4, yellow No. 5, yellow No. 202, yellow No. 203, yellow No. 204, Blue No. 1, Blue No. 2, Blue No. 201, green No. 3, green No. 201, green No. 204, green No. 205, orange No. 201, orange No. 206, orange No. 207 etc.), lakes made from natural dye (carminic acid, laccaic acid, carthamin, brazilin, crocin etc.), and synthetic resin powders made by combining these powders and the like.

Examples of a pearl pigment include titanium oxide coated fluorphlogopite, titanium oxide coated mica, bismuth oxychloride, titanium oxide coated bismuth oxychloride, titanium oxide coated talc, fish scale foil, titanium oxide coated colored mica, titanium oxide coated colored fluorphlogopite and the like.

Examples of a metal powder pigment include aluminum powder, copper powder, stainless powder and the like.

Examples of a powder to be used as a UV scattering agent include white pigments such as titanium oxide, zinc oxide and the like, fine particle powders such as titanium oxide fine particles, cerium oxide fine particles, zinc oxide fine particles and the like, and other fine particles.

The powder to be the core is preferably one or more kinds selected from inorganic powders, and more preferably one or more kinds selected from titanium oxide, zinc oxide, talc, mica, fluorphlogopite and silica.

The shape of the powder to be the core is not particularly limited and may be spherical, nearly spherical, plate or the like.

The plate is a shape having a high aspect ratio (ratio of average particle size and particle thickness), or a layer-like shape. Examples thereof include synthetic mica, plate aluminum oxide flake, silicon dioxide flake, glass flake, perlite flake and the like.

In the present invention, the powder to be the core is preferably a plate. The nearly spherical here refers to those having an aspect ratio of 2.0 or below.

The powder to be the core in the present invention is modified by using the powder modifying agent of the present invention by the above-mentioned modification method. Therefore, the composite powder of the present invention contains N^{ε}-octanoyl-L-lysine being in contact with the powder to be the core.

The content of N^{ε}-octanoyl-L-lysine in the composite powder of the present invention varies depending on the modification method. It is preferably 0.1 mass % - 50 mass %, more preferably 0.2 mass % - 30 mass %, further preferably 0.5 mass % - 15 mass %, with respect to the total mass of the composite powder.

The average particle size of the composite powder of the present invention is generally 0.001 µm - 400 µm, preferably 0.01 - 400 µm, and the lower limit thereof is more preferably 0.1 µm, further preferably 1 µm, further more preferably 10 µm. On the other hand, the upper limit is more preferably 200 µm, further preferably 100 µm, further more preferably 50 µm.

The average particle size of the above-mentioned composite powder can be measured by a laser diffraction scattering method based on Mie scattering theory. To be specific, it can be measured by creating particle size distribution of the composite powder based on volume with a laser scattering particle size distribution analyzer, and taking the median diameter thereof as the average particle size. As the measurement sample, a composite powder dispersed in water by ultrasonication can be preferably used. As the laser scattering particle size distribution analyzer, "LA-950" manufactured by Horiba, Ltd. or the like can be used.

The specific surface area of the composite powder of the present invention is preferably 0.1 m²/g - 1000 m²/g and the lower limit thereof is more preferably 0.5 m²/g, further preferably 1.0 m²/g. On the other hand, the upper limit thereof is more preferably 700 m²/g, further preferably 400 m²/g.

The specific surface area of the composite powder can be measured by the BET method. To be specific, molecules having a known adsorption occupation area are adsorbed on the composite powder sample at the temperature of liquid nitrogen, and the specific surface area of the composite powder sample can be determined from the adsorption amount thereof. As the molecule having a known adsorption occupation area, an inert gas such as nitrogen, helium or the like is preferably used.

The specific surface area of the composite powder can be measured using an automatic specific surface area measuring device. Examples of the automatic specific surface area measuring device include "Macsorb HM-1210" manufactured by MOUNTECH Co., Ltd.

The powder to be the core may be subjected to a hydrophobic surface treatment such as an organosiloxane treatment (e.g., methylhydrogenpolysiloxane treatment, silicone resin treatment, silicone gum treatment, acrylicsilicone treatment, fluorinated silicone treatment and the like), a metal soap treatment (e.g., zinc stearate treatment, acylated amino acid metal salt treatment and the like), a silane treatment (e.g., silane coupling agent treatment, alkylsilane treatment and the like), an organic titanate treatment, an organic aluminate treatment, a fluorine compound treatment (e.g., perfluoroalkylsilane treatment, perfluoroalkyl phosphate treatment, perfluoro polyether treatment and the like), an amino acid treatment (e.g., N-lauroyl-L-lysine treatment and the like), an oil agent treatment (e.g., squalane treatment and the like), a polyacryl ester treatment (e.g., polyacrylic acid methyl treatment and the like) and the like; a hydrophilic surface treatment such as a polyethylene glycol (PEG) silane coupling agent treatment, an agar treatment, a deoxyribonucleic acid treatment, a lecithin treatment, a polyacrylic acid treatment, a silica treatment, an alumina treatment, a zirconia treatment, particularly preferably a cellulose treatment and the like; or other treatment.

A treatment as mentioned above can also be applied to the composite powder of the present invention.

### <makeup cosmetic>

The present invention provides a makeup cosmetic containing the above-mentioned composite powder of the present invention.

The makeup cosmetics of the present invention can be used for the purpose of preparing the skin color, concealing the defects of the skin, improving the condition of the skin, for shielding UV, or for coloring on the face and lips and applying beautiful makeup. The cosmetics can take various forms such as solution, suspension, emulsion, ointment, cream, powder or solid form or the like, preferably the form of ointment, cream, powder or solid, containing a suitable amount of powder.

Therefore, the makeup cosmetics of the present invention is preferably provided as foundation primer such as makeup base cream and the like; make-up cosmetics such as oily ointment type foundation, oil-in-water or water-in-oil emulsion type creamy foundation, solid (cake-type) foundation, concealer, stick-type lip rouge, solid cheek color, ointment-type cheek color, stick-type cheek color, ointment-type eye color, stick-type eye color, solid eye color, solid eyeliner, solid mascara, stick-type eyebrow, solid eyebrow, face powder, pressed powder and the like; and the like.

The makeup cosmetics of the present invention generally contains 0.1 mass % - 50 mass %, preferably 0.2 mass % - 30 mass %, more preferably 0.5 mass % - 15 mass %, of the above-mentioned composite powder of the present invention.

Where necessary, the makeup cosmetics of the present invention can contain, in addition to the composite powder of the present invention, oil agents such as fats and oils (e.g., olive oil, castor oil, coconut oil, cacao butter etc.), wax (e.g., Carnauba wax, candelilla wax, jojoba oil, beeswax, lanolin etc.), hydrocarbon (e.g., squalane, pristine, mineral oil, liquid paraffin, ceresin, microcrystalline wax, petrolatum etc.), fatty acid (e.g., myristic acid, palmitic acid, stearic acid, oleic acid etc.), higher alcohol (e.g., cetanol, stearyl alcohol, cetearyl alcohol, octyldodecanol etc.), ester (e.g., isopropyl myristate, isopropyl palmitate, cetyl 2-ethylhexanoate, myristic acid octyldodecyl ester, diisopropyl sebacate, lauroyl glutamate diisostearyl, lauroyl glutamate di(phytosteryl/octyldodecyl) etc.), silicone oil (e.g., octamethyltrisiloxane, methylhydrogenpolysiloxane, dimethylpolysiloxane, methylphenylpolysiloxane, decamethylcyclopentasiloxane etc.) and the like; solvents such as water, ethanol and the like; polyhydric alcohols such as propanediol, 1,3-butyleneglycol, pentyleneglycol, glycerol, sorbitol and the like; surfactants such as nonionic surfactants (e.g., glyceryl stearate, sorbitan sesquioleate, polyoxyethylene cetyl ether, polyethylene glycol monostearate, polyoxyethylene sorbitan monostearate, polyoxyethylene sorbitan monooleate etc.), anionic surfactants (e.g., sodium lauroylmethyl-β-alanine, sodium lauroyl sarcosine, sodium stearoyl glutamate, polyoxyethylenelauryl ether sodium sulfate etc.), cationic surfactants (e.g., distearyl dimethyl ammonium chloride, stearyl trimethyl ammonium chloride etc.), amphoteric surfactants (e.g., hydrolyzed collagen/resin acid condensate etc.), silicone-based surfactants (e.g., polyoxyethylene/methylpolysiloxane copolymer etc.) and the like; oil gelatinization agent (e.g., dibutyl lauroyl glutamide, N-2-ethylhexanoyl-L-glutamic acid dibutylamide etc.); oil dispersing agents such as organic modified hectorite (e.g., dimethyl distearyl ammonium hectorite etc.), organic modified bentonite (e.g., dimethyl distearyl ammonium bentonite etc.); polymers (e.g., vinyl acetate/vinylpyrrolidone copolymer, polyvinylpyrrolidone, trimethylsilylpullulan etc.); metal soap (e.g., magnesium stearate, zinc stearate etc.), organic powders such as resin powders (e.g., nylon powder, crosslinking-type silicone powder etc.) and the like; humectants (e.g., panthenol, cholesterol etc.); anti-inflammatory agents (e.g., disodium succinyl glycyrrhetinate, oryzanol, bisabolol etc.); ultraviolet absorbers (e.g., 2-ethylhexyl salicylate, homomenthyl salicylate, 2-ethylhexyl paramethoxycinnamate, oxybenzone, hydroxy methoxy benzophenone sulfonate, 4-tert-butyl-4'-methoxydibenzoylmethane etc.); amino acids (e.g., alanine, arginine, glycine, glutamic acid, proline etc.); polyamino acid and a salt thereof (e.g., sodium poly(aspartate) etc.); vitamins such as vitamin A (e.g., retinol, retinyl palmitate etc.), vitamin Bs (e.g., calcium pantothenate etc.), vitamin C (e.g., sodium L-ascorbate, phosphate L-ascorbylmagnesium etc.), vitamin D (e.g., cholecalciferol etc.), vitamin E (e.g., d-δ-tocopherol, dl-α-tocopherol etc.) and the like; preservatives (e.g., sodium benzoate, phenoxyethanol, methyl paraoxybenzoate, propyl paraoxybenzoate etc.); antioxidants (e.g., tocopheryl acetate, gallic acid, dipalmitoylhydroxyproline etc.); pH adjusters (e.g., hydrochloric acid, lactic acid, citric acid, sodium hydroxide, potassium hydroxide etc.); colorants such as natural dyes (e.g., β-carotene, rutin etc.), tar pigments (e.g., Red No. 202, Blue No. 404 etc.) and the like; flavors (e.g., menthol, peppermint oil etc.) and the like.

The makeup cosmetics of the present invention can be produced according to the form thereof, dosage form thereof and the like by a method well known to those of ordinary skill in the art.

Oily ointment-type makeup cosmetics can be produced, for example, as follows.

First, other powder components are added as necessary to the composite powder of the present invention and mixed. Base components such as an oil agent, a preservative, an antioxidant and the like are separately mixed and made uniform by heating melting. Thereto are added the aforementioned powder components and the mixture is kneaded by a kneader such as a roll mill and the like. Then the kneaded mixture is remelted, toned, deaerated by slowly stirring the mixture, and cooled with stirring. A flavor is added at 60°C, and the mixture is poured into a container and allowed to cool for solidification.

Creamy makeup cosmetics can be produced, for example, as follows.

First, aqueous phase components are mixed, dissolved by heating to the uniformity of the mixture, and the mixture is heated to 75°C. Separately, oil phase components are mixed and made uniform by heating melting, the composite powder of the present invention is added and dispersed and the mixture is set to 80°C. To the aforementioned aqueous phase is added the aforementioned oil phase with stirring to allow for emulsification (oil-in-water type), or to the aforementioned oil phase is added the aforementioned aqueous phase with stirring to allow for emulsification (water-in-oil type). The emulsion is cooled by stirring, a flavor is added at 50°C, and cooled to room temperature by stirring further.

Solid (cake-type) makeup cosmetics can be produced, for example, as follows.

Other powder components and a colorant are added as necessary to the composite powder of the present invention, is added, mixed and pulverized. An oil agent as a binder, a surfactant and the like are mixed with a preservative, a flavor and the like, and the mixture is added to the aforementioned powder component and uniformly mixed. This is treated in a grinding machine, the particle sizes are adjusted by passing through a sieve, filled in a container such as a metal tray and the like and compression molded.

Powdery makeup cosmetics can be produced, for example, as follows.

The composite powder of the present invention containing an extender such as talc and the like and a coloration pigment such as iron oxide and the like as the inorganic powder (C) is mixed in a blender, other additional components such as magnesium stearate and the like as a lubricant and the like are added, and the mixture is toned and uniformly mixed with spray of a flavor. This is passed through a grinding machine to allow for pulverization, passed through a sieve and filled in a container.

Stick-type makeup cosmetics can be produced, for example, as follows.

Base components such as an oil agent, an antioxidant, a preservative and the like are melted by heating, and uniformly mixed. Thereto are added the composite powder of the present invention and a colorant, kneaded in a kneader such as a roll mill and the like and uniformly dispersed. Then, the mixture is remelted, a flavor is added, and the mixture is defoamed, poured into a mold, and rapidly cooled to allow for solidification. The solidified product is taken out from the mold, filled in a container and subjected to a framing treatment as necessary.

Since the makeup cosmetic of the present invention contains the above-mentioned composite powder of the present invention, it is superior in the texture when applied to the skin (smoothness, moist feeling etc.), and water-repellency, adhesiveness and transparency.

### [Example]

The present invention is explained in detail based on the following Examples. In the following, "%" means "mass %" unless particularly indicated.

### [Example 1] synthetic mica modified with 10% N^{ε}-octanoyl-L-lysine aqueous solution

150 g of synthetic mica and 1350 g of deionized water were charged in a 5 L flask and the mixture was stirred at a stirring rate of 600 rpm. Then, 32% calcium chloride aqueous solution (24.6 g) was added, and the mixture was stirred for 30 min. Thereafter, 10% N^{ε}-octanoyl-L-lysine aqueous solution (1365 g) prepared using 3.5% aqueous sodium hydroxide solution was added dropwise while maintaining the pH of the aforementioned synthetic mica dispersion at 5.8 - 6.2 with 10% hydrochloric acid. The rate of dropwise addition of 10% N^{ε}-octanoyl-L-lysine aqueous solution and 10% hydrochloric acid was about 5 g/min. After dropwise addition of 10% N^{ε}-octanoyl-L-lysine aqueous solution, the pH of the dispersion was adjusted to 7.

A slurry of the obtained composite powder was filtered and washed with 3000 mL of deionized water (6 times with 500 mL/washing). The washed composite powder was dried at 120°C for about 3 hr until it reached a given weight. After drying, the composite powder was sieved with #200 filter net and used as Example 1.

### [Examples 2, 3] synthetic mica modified with N^{ε}-octanoyl-L-lysine aqueous solution at each concentration of 4.5% or 7.5%

In substantially the same manner as in Example 1, synthetic mica was modified with 4.5% or 7.5% N^{ε}-octanoyl-L-lysine aqueous solution. When modified with 4.5% aqueous solution, N^{ε}-octanoyl-L-lysine aqueous solution was added dropwise, pH of the dispersion was adjusted to 7, and the composite powder was filtered and washed. The composite powder modified with 4.5% aqueous solution was dried at 110°C for about 2 hr until it reached a given weight.

The composite powder of synthetic mica modified with 4.5% N^{ε}-octanoyl-L-lysine aqueous solution was used as Example 2, and the composite powder modified with 7.5% N^{ε}-octanoyl-L-lysine aqueous solution was used as Example 3.

### [Example 4] titanium oxide modified with 10% N^{ε}-octanoyl-L-lysine aqueous solution

The composite powder obtained in the same manner as in Example 1 except that titanium oxide was used instead of synthetic mica was used as Example 4.

### [Examples 5, 6] titanium oxide modified with N^{ε}-octanoyl-L-lysine aqueous solution at each concentration of 4.5% or 7.5%

In the same manner as in Examples 2, 3 except that titanium oxide was used instead of synthetic mica, modification was performed with N^{ε}-octanoyl-L-lysine aqueous solution at each concentration of 4.5% or 7.5% and the obtained composite powders were respectively used as Example 5 and Example 6.

[Example 7] talc modified with 10% N^{ε}-octanoyl-L-lysine aqueous solution

The composite powder obtained in the same manner as in Example 1 except that talc was used instead of synthetic mica was used as Example 7.

### [Examples 8, 9] talc modified with N^{ε}-octanoyl-L-lysine aqueous solution at each concentration of 4.5% or 7.5%

In the same manner as in Examples 2, 3 except that talc was used instead of synthetic mica, modification was performed with N^{ε}-octanoyl-L-lysine aqueous solution at each concentration of 4.5% or 7.5% and the obtained composite powders were respectively used as Example 8 and Example 9.

### [Example 10] zinc oxide modified with 10% N^{ε}-octanoyl-L-lysine aqueous solution

The composite powder obtained in the same manner as in Example 1 except that zinc oxide was used instead of synthetic mica was used as Example 10.

### [Examples 11, 12] zinc oxide modified with N^{ε}-octanoyl-L-lysine aqueous solution at each concentration of 4.5% or 7.5%

In the same manner as in Examples 2, 3 except that zinc oxide was used instead of synthetic mica, modification was performed with N^{ε}-octanoyl-L-lysine aqueous solution at each concentration of 4.5% or 7.5% and the obtained composite powders were respectively used as Example 11 and Example 12.

### [Example 13] silicone resin powder modified with 10% N^{ε}-octanoyl-L-lysine aqueous solution

The composite powder obtained in the same manner as in Example 1 except that silicone resin powder was used instead of synthetic mica was used as Example 13.

### [Examples 14, 15] silicone resin powder modified with N^{ε}-octanoyl-L-lysine aqueous solution at each concentration of 4.5% or 7.5%

In the same manner as in Examples 2, 3 except that silicone resin powder was used instead of synthetic mica, modification was performed with N^{ε}-octanoyl-L-lysine aqueous solution at each concentration of 4.5% or 7.5% and the obtained composite powders were respectively used as Example 14 and Example 15.

### [Example 16] red iron oxide modified with 10% N^{ε}-octanoyl-L-lysine aqueous solution

The composite powder obtained in the same manner as in Example 1 except that red iron oxide was used instead of synthetic mica was used as Example 16.

### [Example 17] yellow iron oxide modified with 10% N^{ε}-octanoyl-L-lysine aqueous solution

The composite powder obtained in the same manner as in Example 1 except that yellow iron oxide was used instead of synthetic mica was used as Example 17.

### [Example 18] black iron oxide modified with 10% N^{ε}-octanoyl-L-lysine aqueous solution

The composite powder obtained in the same manner as in Example 1 except that black iron oxide was used instead of synthetic mica was used as Example 18.

### [Example 19] sericite modified with 10% N^{ε}-octanoyl-L-lysine aqueous solution

The composite powder obtained in the same manner as in Example 1 except that sericite was used instead of synthetic mica was used as Example 19.

### [Example 20] mica modified with 10% N^{ε}-octanoyl-L-lysine aqueous solution

The composite powder obtained in the same manner as in Example 1 except that mica was used instead of synthetic mica was used as Example 20.

### [Example 21] titanium oxide modified with formation of coating film containing N^{ε}-octanoyl-L-lysine and stearic acid

N^{ε}-octanoyl-L-lysine (3.3 g) and stearic acid (1.0 g) were dissolved in 1% alkali aqueous solution. A starting material powder, titanium oxide (100 g) was added and suspended in the obtained solution (titanium oxide content=20%) and the mixture was stirred for 30 min. To the suspension was slowly added dropwise a 10% aqueous solution of magnesium chloride hexahydrate (0.7 g). Then, the mixture was neutralized with hydrochloric acid, and the mixture was stirred for 30 min more, repeatedly filtered and washed with water and dried at 80°C for 30 hr. The dried product was pulverized to give a composite powder of titanium oxide with a coating film containing N^{ε}-octanoyl-L-lysine and stearic acid formed thereon.

### [Example 22] sericite modified with formation of coating film containing N^{ε}-octanoyl-L-lysine and stearic acid

In the same manner as in Example 21 except that sericite was used instead of titanium oxide, a composite powder of sericite with a coating film containing N^{ε}-octanoyl-L-lysine and stearic acid formed thereon was obtained.

### [Example 23] titanium oxide modified with formation of coating film containing N^{ε}-octanoyl-L-lysine and phosphate having perfluoroalkyl group

Ion exchange water (1000 parts by mass)) and titanium oxide (100 parts by mass)) were mixed and stirred to disperse titanium oxide. Then, an aqueous solution of diethanolamine salt of perfluoroalkyl phosphate ("AG-530", manufactured by ASAHI GLASS CO., LTD., 5 parts by mass) diluted about 20-fold was added dropwise, after which hydrochloric acid aqueous solution diluted appropriately was slowly added dropwise until the pH of the aforementioned titanium oxide dispersion became not more than 3. After heating the aqueous solution to 80°C, and a water dispersion of ion exchange water (100 parts by mass)) and N^{ε}-octanoyl-L-lysine (2 parts by mass) heat dispersed in advance at pH=3 or below was added and the mixture was stirred well. Thereafter, the mixture was neutralized with aqueous sodium carbonate solution until the pH of titanium oxide dispersion became 6. Then, the treated powder was washed several times with water, filtered, dried and pulverized to give a composite powder of titanium oxide with a coating film containing N^{ε}-octanoyl-L-lysine and phosphate having a perfluoroalkyl group formed thereon.

### [Example 24] sericite modified with formation of coating film containing N^{ε}-octanoyl-L-lysine and phosphate having perfluoroalkyl group

In the same manner as in Example 23 except that sericite was used instead of titanium oxide, a composite powder of sericite with a coating film containing N^{ε}-octanoyl-L-lysine and phosphate having a perfluoroalkyl group formed thereon was obtained.

### [Example 25] talc modified with formation of coating film containing N^{ε}-octanoyl-L-lysine and phosphate having perfluoroalkyl group

In the same manner as in Example 23 except that talc was used instead of titanium oxide, a composite powder of talc with a coating film containing N^{ε}-octanoyl-L-lysine and phosphate having a perfluoroalkyl group formed thereon was obtained.

### [Example 26] mica modified with formation of coating film containing N^{ε}-octanoyl-L-lysine and phosphate having perfluoroalkyl group

In the same manner as in Example 23 except that mica was used instead of titanium oxide, a composite powder of mica with a coating film containing N^{ε}-octanoyl-L-lysine and phosphate having a perfluoroalkyl group formed thereon was obtained.

### [Examples 27 - 33] pigment powder modified with N^{ε}-octanoyl-L-lysine and n-octyltriethoxysilane

Ion exchange water (4 kg), sodium hydroxide (60 g) and N^{ε}-octanoyl-L-lysine (52.7 g) were mixed and stirred, heated to 80°C and titanium oxide (1 kg) was added. Then, sulfuric acid aqueous solution diluted appropriately was slowly added dropwise until the pH of the aforementioned titanium oxide dispersion became not more than 3, and the mixture was stirred sufficiently. Thereafter, n-octyltriethoxysilane (20.4 g) was added, the mixture was sufficiently stirred and neutralized with aqueous sodium carbonate solution until the pH of titanium oxide dispersion became 6. Then, the treated powder was washed several times with water, filtered, dried and pulverized to give a composite powder of titanium oxide with the surface coated with N^{ε}-octanoyl-L-lysine and n-octyltriethoxysilane which was used as Example 27.

By a method similar to that in Example 27, the composite powder obtained using sericite instead of titanium oxide was used as Example 28, the composite powder obtained using mica was used as Example 29, the composite powder obtained using talc was used as Example 30, the composite powder obtained using red iron oxide was used as Example 31, the composite powder obtained using yellow iron oxide was used as Example 32, and the composite powder obtained using black iron oxide was used as Example 33.

### [Examples 34 - 40] pigment powder modified with N^{ε}-octanoyl-L-lysine and isopropyl triisostearoyl titanate

A composite powder with the surface coated with N^{ε}-octanoyl-L-lysine and isopropyl triisostearoyl titanate was obtained by a similar to that in Example 27 except that isopropyl triisostearoyl titanate (20.4 g) was used instead of n-octyltriethoxysilane and used as Example 34.

In addition, a composite powder obtained by a method similar to that in Example 34 except that sericite was used instead of titanium oxide was used as Example 35, a composite powder obtained using mica was used as Example 36, a composite powder obtained using talc was used as Example 37, a composite powder obtained using red iron oxide was used as Example 38, a composite powder obtained using yellow iron oxide was used as Example 39, and a composite powder obtained using black iron oxide was used as Example 40.

### [Examples 41 - 47] pigment powder modified with N^{ε}-octanoyl-L-lysine and n-octyltriethoxysilane

In a stainless reaction container provided with a stirring and heating device, ion exchange water (4 kg), sodium hydroxide (60 g) and N^{ε}-octanoyl-L-lysine (52.7 g) were placed, stirred and heated to 80°C and titanium oxide (1 kg) was added. Then, sulfuric acid aqueous solution diluted appropriately was slowly added dropwise until the pH of the aforementioned titanium oxide dispersion became not more than 3, and the mixture was stirred at 80°C for 1 hr. Thereafter, the mixture was neutralized with aqueous sodium carbonate solution until the pH of titanium oxide dispersion became 6, and the treated powder was washed several times with water and filtered. Then, the recovered powder was transferred into a Henschel mixer, water (400 g) was added and stirred, n-octyltriethoxysilane (20.4 g) was added with stirring and mixed well. Thereafter, heat was applied to the Henschel mixer, the pressure in the Henschel mixer was reduced and water was removed. Then, the treated powder was taken out from the Henschel mixer, heated in a dryer, and pulverized to give a composite powder of titanium oxide with the surface coated with N^{ε}-octanoyl-L-lysine and n-octyltriethoxysilane which was used as Example 41.

By a method similar to that in Example 41, a composite powder obtained using sericite instead of titanium oxide was used as Example 42, a composite powder obtained using mica was used as Example 43, a composite powder obtained using talc was used as Example 44, a composite powder obtained using red iron oxide was used as Example 45, a composite powder obtained using yellow iron oxide was used as Example 46, and a composite powder obtained using black iron oxide was used as Example 47.

### [Examples 48 - 57] composite powder modified with N^{ε}-octanoyl-L-lysine and N^{ε}-lauroyl-L-lysine

The composite powders obtained by a similar treatment in Examples 1, 4, 7, 10, 13, 16 - 20 and using a mixed aqueous solution of N^{ε}-octanoyl-L-lysine and N^{ε}-lauroyl-L-lysine (content ratio of N^{ε}-octanoyl-L-lysine and N^{ε}-lauroyl-L-lysine = 1:1 (mass ratio), total content=10%) (1365 g) instead of 10% N^{ε}-octanoyl-L-lysine aqueous solution (1365 g) were used as Examples 48 - 57.

### [Example 58] synthetic mica modified with N^{ε}-octanoyl-L-lysine and N^{ε}-lauroyl-L-lysine

150 g of synthetic mica and 1350 g of deionized water were charged in a 5 L flask and the mixture was stirred at a stirring rate of 600 rpm. Then, 32% calcium chloride aqueous solution (24.6 g) was added, and the mixture was stirred for 30 min. Thereafter, 10% N^{ε}-octanoyl-L-lysine aqueous solution (623 g) prepared using 3.5% aqueous sodium hydroxide solution was added dropwise while maintaining the pH of the aforementioned synthetic mica dispersion at 5.8 - 6.2 with 10% hydrochloric acid. The rate of dropwise addition of 10% N^{ε}-octanoyl-L-lysine aqueous solution and 10% hydrochloric acid was about 5 g/min. After dropwise addition of 10% N^{ε}-octanoyl-L-lysine aqueous solution, the pH of the synthetic mica dispersion was adjusted to 7. Then, 10% N^{ε}-lauroyl-L-lysine aqueous solution (623 g) prepared using 5% aqueous sodium hydroxide solution was added dropwise while maintaining the pH of the synthetic mica dispersion at 5.8 - 6.2 with 10% hydrochloric acid. The rate of dropwise addition of 10% N^{ε}-lauroyl-L-lysine aqueous solution and 10% hydrochloric acid was about 5 g/min. After dropwise addition of 10% N^{ε}-octanoyl-L-lysine aqueous solution, the pH of the synthetic mica dispersion was adjusted to 7.

A slurry of the treated powder was filtered and washed with 3000 mL of deionized water (6 times with 500 mL/washing). The washed powder was dried at 120°C for about 3 hr until it reached a given weight and sieved with #200 filter net. The composite powder obtained by this step was used as Example 58.

### [Example 59] titanium oxide modified with N^{ε}-octanoyl-L-lysine and N^{ε}-lauroyl-L-lysine

The composite powder obtained by a treatment similar to that in Example 58 except that titanium oxide was used instead of synthetic mica was used as Example 59.

### [Example 60] black iron oxide modified with N^{ε}-octanoyl-L-lysine and N^{ε}-lauroyl-L-lysine

The composite powder obtained by a treatment similar to that in Example 58 except that black iron oxide was used instead of synthetic mica was used as Example 60.

### [Example 61] synthetic mica modified with N^{ε}-lauroyl-L-lysine and N^{ε}-octanoyl-L-lysine

150 g of synthetic mica and 1350 g of deionized water were charged in a 5 L flask and the mixture was stirred at a stirring rate of 600 rpm. Then, 32% calcium chloride aqueous solution (24.6 g) was added, and the mixture was stirred for 30 min. Thereafter, 10% N^{ε}-lauroyl-L-lysine aqueous solution (623 g) prepared using 3.5% aqueous sodium hydroxide solution was added dropwise while maintaining the pH of the aforementioned synthetic mica dispersion at 5.8 - 6.2 with 10% hydrochloric acid. The rate of dropwise addition of 10% N^{ε}-lauroyl-L-lysine aqueous solution and 10% hydrochloric acid was about 5 g/min. After dropwise addition of 10% N^{ε}-lauroyl-L-lysine aqueous solution, the pH of the synthetic mica dispersion was adjusted to 7. Then, 10% N^{ε}-octanoyllauroyl-L-lysine aqueous solution (623 g) prepared using 5% aqueous sodium hydroxide solution was added dropwise while maintaining the pH of the aforementioned dispersion at 5.8 - 6.2 with 10% hydrochloric acid. The rate of dropwise addition of 10% N^{ε}-octanoyl-L-lysine aqueous solution and 10% hydrochloric acid was about 5 g/min. After dropwise addition of 10% N^{ε}-octanoyl-L-lysine aqueous solution, the pH of the synthetic mica dispersion was adjusted to 7.

A slurry of the treated powder was filtered and washed with 3000 mL of deionized water (6 times with 500 mL/washing). The washed powder was dried at 120°C for about 3 hr until it reached a given weight and sieved with #200 filter net. The composite powder obtained by this step was used as Example 61.

### [Example 62] mixed powder of Fe doped titanium oxide fine particles and plate barium sulfate modified with N^{ε}-octanoyl-L-lysine

Fe doped titanium oxide fine particles (average particle size =35 nm, yellow) (20 parts by mass)) and plate barium sulfate (75 parts by mass) were mixed in a mixer. Thereto was added purified water (250 parts by mass) and the mixture was stirred to give slurry A. Then, 10% N^{ε}-octanoyl-L-lysine (5 parts by mass) was dissolved in a mixed solution of 5N sodium hydroxide (13 parts by mass) and purified water (80 parts by mass)) to prepare solution B. Solution B was slowly added dropwise (dropwise addition time = 30 min) while vigorously stirring slurry A. After completion of the dropwise addition, the pH of slurry A was adjusted to 7.0 with 6N hydrochloric acid and the mixture was further stirred for 30 min. The obtained slurry was filtered, purified water was added and desalting was repeated until the sodium chloride concentration measured using a salinometer became 0.01% or below. The obtained treated powder was dried with a airflow dryer at 85°C for 12 hr and heated at 125°C for 5 hr for sterilization. The powder was pulverized with a mixer to give the object modified powder (treatment concentration with 10% N^{ε}-octanoyl-L-lysine=5%) which was used as Example 62.

### [Example 63] titanium oxide, titanyl sulfate-treated N^{ε}-octanoyl-L-lysine

N^{ε}-octanoyl-L-lysine (100 parts by mass) was dispersed in isopropyl alcohol (2000 parts by mass), and 5 parts by mass of titanium tetraisopropoxide, which is one kind of organic titanium, was added to the aforementioned dispersion and the mixture was stirred well. Then, purified water (10 parts by mass) was added and stirring was continued for one day, and then the solvent was removed by heating evaporation under reduced pressure. Then, titanyl sulfate (70 parts by mass) was dissolved in purified water (2000 parts by mass) to give an aqueous solution, the above-mentioned powder was added thereto, the pH of the aforementioned powder dispersion was neutralized by adjusting to 7.0 with sodium hydroxide with stirring. Purified water (3000 parts by mass) was added, and filtration and washing with water were repeated. Washing with water was stopped when the salinity concentration became less than 0.1%, and the obtained treated powder was transferred to a stainless vat and dried at 130°C for 30 hr under ventilation. The dried treated powder was pulverized and passed through a mesh to give the object modified powder and used as Example 63.

### [Example 64] fluorine-treated silica/titanium oxide-treated N^{ε}-octanoyl-L-lysine

N^{ε}-octanoyl-L-lysine (100 parts by mass) was dispersed in isopropyl alcohol (2000 parts by mass), and 5 parts by mass of tetraethoxysilane, which is one kind of organic silicon, was added thereto and the mixture was stirred well. Thereto was added purified water (5 parts by mass) and the mixture was stirred for 6 hr. Titanium tetraisopropoxide (30 parts by mass) was added and purified water (10 parts by mass) was further added. Then, 1 M hydrochloric acid (1 part by mass) was added and the mixture was left standing under stirring for one day. After filtration under reduced pressure in sealed condition, the obtained powder was thoroughly washed with water, neutralized with sodium carbonate water, filtered, washed with water, and dried at 130°C for 36 hours using an airflow dryer. The dried powder was pulverized and passed through a mesh to give a modified powder, silica/titanium oxide-treated N^{ε}-octanoyl-L-lysine. Then, a water base coating treatment utilizing desalting of diethanolamine was performed further using diethanolamine salt of perfluoroalkyl phosphate at a coating amount of 5 parts by mass per 100 parts by mass of the aforementioned modified powder to give fluorine-treated silica/titanium oxide-treated N^{ε}-octanoyl-L-lysine which was used as Example 64.

### [Examples 65 - 69] composite powder modified with cholesteryl/octyldodecyl lauroyl glutamate and N^{ε}-octanoyl-L-lysine

The following modification treatment was performed using each of talc, mica and sericite as a powder to be the core, and each of red iron oxide, yellow iron oxide, black iron oxide and titanium oxide as a color pigment.

The powder to be the core (30 parts by mass) and color pigment (8.0 parts by mass) were dispersed well in purified water (170 parts by mass), 6 mol/L hydrochloric acid (2.8 parts by mass) was added, cholesteryl/octyldodecyl lauroyl glutamate ("Eldew CL-202", manufactured by Ajinomoto Co., Inc.) (4.0 parts by mass) was further added and the mixture was stirred well (for 10 min). Then, N^{ε}-octanoyl-L-lysine (2.0 parts by mass) was dissolved in a mixed solution of 5 mol/L aqueous sodium hydroxide solution (3.6 parts by mass) and purified water (26 parts by mass) and added to the above-mentioned powder dispersion. Using 1 mol/L hydrochloric acid or 1 mol/L aqueous sodium hydroxide solution, the aforementioned powder dispersion was neutralized, filtered and washed with water. The obtained cake was transferred to a metal tray, and dried in a fan drying machine set to 80°C for 24 hr to give the composite powder of the present invention.

A composite powder obtained according to the above-mentioned method and using talc as the powder to be the core and red iron oxide as the color pigment was used as Example 65, a composite powder obtained using talc as the powder to be the core and yellow iron oxide as the color pigment was used as Example 66, a composite powder obtained using talc as the powder to be the core and black iron oxide as the color pigment was used as Example 67, a composite powder obtained using mica as the powder to be the core and titanium oxide as the color pigment was used as Example 68, and a composite powder obtained using sericite as the powder to be the core and titanium oxide as the color pigment was used as Example 69.

### [Examples 70 - 74] composite powder modified with cholesteryl/octyldodecyl lauroyl glutamate and N^{ε}-octanoyl-L-lysine

The following modification treatment was performed using each of talc, mica and sericite as a powder to be the core, and each of red iron oxide, yellow iron oxide, black iron oxide and titanium oxide as a color pigment.

The powder to be the core (30 parts by mass) and color pigment (8.0 parts by mass) were dispersed well in purified water (170 parts by mass), 6 mol/L hydrochloric acid (2.8 parts by mass) was added, cholesteryl/octyldodecyl lauroyl glutamate ("Eldew CL-202", manufactured by Ajinomoto Co., Inc.) (1.0 part by mass) was further added and the mixture was stirred well. Then, N^{ε}-octanoyl-L-lysine (2.0 parts by mass) was dissolved in a mixed solution of 5 mol/L aqueous sodium hydroxide solution (3.6 parts by mass) and purified water (26 parts by mass). The prepared aqueous solution was divided equally into four and one part thereof was added to the above-mentioned powder dispersion. Successively, cholesteryl/octyldodecyl lauroyl glutamate ("Eldew CL-202", manufactured by Ajinomoto Co., Inc.) (1.0 part by mass) was added, and the mixture was stirred well. An alkali aqueous solution of N^{ε}-octanoyl-L-lysine divided equally into four earlier was added and this operation was repeated two more times. Using 1 mol/L hydrochloric acid or 1 mol/L aqueous sodium hydroxide solution, the aforementioned powder dispersion was neutralized, filtered and washed with water. The obtained cake was transferred to a metal tray, and dried in a fan drying machine set to 80°C for 24 hr to give the composite powder of the present invention.

A composite powder obtained according to the above-mentioned method and using talc as the powder to be the core and red iron oxide as the color pigment was used as Example 70, a composite powder obtained using talc as the powder to be the core and yellow iron oxide as the color pigment was used as Example 71, a composite powder obtained using talc as the powder to be the core and black iron oxide as the color pigment was used as Example 72, a composite powder obtained using mica as the powder to be the core and titanium oxide as the color pigment was used as Example 73, and a composite powder obtained using sericite as the powder to be the core and titanium oxide as the color pigment was used as Example 74.

### [Examples 75 - 78] composite powder modified with cholesteryl/octyldodecyl lauroyl glutamate and N^{ε}-octanoyl-L-lysine

The following modification treatment was performed using each of talc and mica as a powder to be the core, and each of red No. 226, yellow No. 401, ultramarine and titanium oxide as a color pigment.

The powder to be the core (34 parts by mass) and colorant (4.0 parts by mass) were dispersed well in purified water (170 parts by mass), 6 mol/L hydrochloric acid (2.8 parts by mass) was added, cholesteryl/octyldodecyl lauroyl glutamate ("Eldew CL-202", manufactured by Ajinomoto Co., Inc.) (4.0 part by mass) was further added and the mixture was stirred well. Then, N^{ε}-octanoyl-L-lysine (2.0 parts by mass) was dissolved in a mixed solution of 5 mol/L aqueous sodium hydroxide solution (3.6 parts by mass) and purified water (26 parts by mass) and the aqueous solution was added to the above-mentioned powder dispersion. Using 1 mol/L hydrochloric acid or 1 mol/L aqueous sodium hydroxide solution, the aforementioned powder dispersion was neutralized, filtered and washed with water. The obtained cake was transferred to a metal tray, and dried in a fan drying machine set to 80°C for 24 hr to give the composite powder of the present invention.

A composite powder obtained according to the above-mentioned method and using talc as the powder to be the core and red No. 226 as the colorant was used as Example 75, a composite powder obtained using talc as the powder to be the core and yellow No. 401 as the colorant was used as Example 76, a composite powder obtained using talc as the powder to be the core and ultramarine as the colorant was used as Example 77, and a composite powder obtained using mica as the powder to be the core and titanium oxide as the colorant was used as Example 78.

### [Example 79] titanium oxide fine particle-zinc oxide fine particle mixed powder modified with sodium zinc cetyl phosphate and N^{ε}-octanoyl-L-lysine

A mixture of titanium oxide fine particles (average particle size=0.05 µm) and zinc oxide fine particles (average particle size=0.01 µm) (10 g) (mixing mass ratio=1:1) was dispersed in water (200 mL) by a wet medium dispersing machine, sodium zinc cetyl phosphate (average particle size: major axis 16 µm, minor axis 13 µm, thickness 0.8 µm) (50 g) was added, and the mixture was dispersed by stirring at room temperature for 1 hr, filtered and washed twice with water. This was dried and pulverized to give composite powder (1) (58 g) modified with sodium zinc cetyl phosphate.

In another round-bottle flask, 100 mL of purified water and 5 g of sodium hydroxide were added and dissolved, and then N^{ε}-octanoyl-L-lysine (7.5 g) was added and dissolved at 80°C. On the other hand, the aforementioned composite powder (1) (50 g) was added to acidic water made of purified water (200 mL) and 35% hydrochloric acid (6 mL) and dispersed for 1 hr. The aforementioned N^{ε}-octanoyl-L-lysine aqueous solution was added dropwise over 30 min. The aforementioned powder dispersion was neutralized, further mixed by stirring for 1 hr, washed with water, filtered, dried and pulverized to give titanium oxide fine particles-zinc oxide fine particles mixed powder (52 g) modified with sodium zinc cetyl phosphate and N^{ε}-octanoyl-L-lysine.

### [Example 80] composite powder modified with fluorine compound and N^{ε}-octanoyl-L-lysine

The composite powder (50 g) modified with N^{ε}-octanoyl-L-lysine in Example 93 was placed in a round-bottle flask, ion exchange water (500 mL) was added, a 15% aqueous solution (15 g) of dioxyethylamine salt of perfluoroalkyl phosphate (carbon number of perfluoroalkyl group = 6 - 18, average carbon number = 9) was added and the mixture was stirred at 60°C for 2 hr. Then, 35% hydrochloric acid was added to lower the pH of the aqueous solution to 3, and the mixture was further stirred at 80°C for 1 hr. This was neutralized with 5% sodium carbonate, filtered, washed with water, dried and pulverized to give a composite powder (51 g) modified with fluorine compound and N^{ε}-octanoyl-L-lysine which was used as Example 80.

### [Example 81] titanium oxide fine particles modified with fluorine compound and N^{ε}-octanoyl-L-lysine

Titanium oxide fine particles (average particle size=0.05 µm) (12 g) and N^{ε}-octanoyl-L-lysine (48 g) were mixed and pulverized in a pulverizer for analysis(manufactured by Nippon Rikagaku Kikai) to give composite powder (1) (56 g).

In another round-bottle flask, 100 mL of purified water and 5 g of sodium hydroxide were added and dissolved, and then N^{ε}-octanoyl-L-lysine (7.5 g) was added and dissolved at 80°C. On the other hand, the aforementioned composite powder (1) (50 g) was added to acidic water made of purified water (200 mL) and 35% hydrochloric acid (6 mL) and dispersed for 1 hr. The aforementioned N^{ε}-octanoyl-L-lysine aqueous solution was added dropwise over 30 min to the dispersion. The aforementioned dispersion was neutralized, further mixed by stirring for 1 hr, washed with water, filtered, dried and pulverized to give composite powder (2) (52 g) modified with N^{ε}-octanoyl-L-lysine.

The composite powder (2) (50 g) modified with the above-mentioned N^{ε}-octanoyl-L-lysine was placed in a round-bottle flask, ion exchange water (500 mL) was added, a 15% aqueous solution (15 g) of dioxyethylamine salt of perfluoroalkyl phosphate (carbon number of perfluoroalkyl group = 6 - 18, average carbon number = 9) was added and the mixture was stirred at 60°C for 2 hr. Then, 35% hydrochloric acid was added to lower the pH of the aforementioned dispersion to 3, and the mixture was further stirred at 80°C for 1 hr. This was neutralized with 5% sodium carbonate, filtered, washed with water, dried and pulverized to give titanium oxide fine particles (51 g) modified with fluorine compound and N^{ε}-octanoyl-L-lysine which was used as Example 81.

### [Examples 82 - 87] powder modified with cholesteryl/octyldodecyl lauroyl glutamate, phytosteryl/octyldodecyl lauroyl glutamate and N^{ε}-octanoyl-L-lysine

The following modification treatment was performed using red iron oxide, yellow iron oxide, black iron oxide, titanium oxide, mica titanium and iron blue-coated mica titanium.

The above-mentioned powders (38 parts by mass) were each dispersed well in purified water (250 parts by mass), 6 mol/L hydrochloric acid (2.8 parts by mass) was added, cholesteryl/octyldodecyl lauroyl glutamate ("Eldew CL-202", manufactured by Ajinomoto Co., Inc.) (0.4 parts by mass) was further added and the mixture was stirred well. Then, N^{ε}-octanoyl-L-lysine (2.0 parts by mass) was dissolved in a mixed solution of 5 mol/L aqueous sodium hydroxide solution (3.6 parts by mass) and purified water (26 parts by mass). The prepared aqueous solution was divided equally into four and one part thereof was added to the above-mentioned powder dispersion. Successively, phytosteryl/octyldodecyl lauroyl glutamate ("Eldew PS-203", manufactured by Ajinomoto Co., Inc.) (0.2 parts by mass) was added, and the mixture was stirred well, alkali aqueous solution of N^{ε}-octanoyl-L-lysine divided equally into four earlier was added and this operation was repeated two more times. Using 1 mol/L hydrochloric acid or 1 mol/L aqueous sodium hydroxide solution, the aforementioned powder dispersion was neutralized, filtered and washed with water. The obtained cake was transferred to a metal tray, and dried in a fan drying machine set to 80°C for 24 hr to give the composite powder of the present invention.

A composite powder obtained according to the above-mentioned method and using red iron oxide was used as Example 82, a composite powder obtained using yellow iron oxide was used as Example 83, a composite powder obtained using black iron oxide was used as Example 84, a composite powder obtained using titanium oxide was used as Example 85, a composite powder obtained using mica titanium was used as Example 86, and a composite powder obtained using iron blue-coated mica titanium was used as Example 87.

### [Example 88] plate barium sulfate modified with phytosteryl/octyldodecyl lauroyl glutamate and N^{ε}-octanoyl-L-lysine

Plate barium sulfate (36 parts by mass) was dispersed well in purified water (160 parts by mass), 6 mol/L hydrochloric acid (5.6 parts by mass) was added, phytosteryl/octyldodecyl lauroyl glutamate ("Eldew PS-203", manufactured by Ajinomoto Co., Inc.) (0.3 parts by mass) was further added and the mixture was stirred well. Then, N^{ε}-octanoyl-L-lysine (4.0 parts by mass) was dissolved in a mixed solution of 5 mol/L aqueous sodium hydroxide solution (7.2 parts by mass) and purified water (26 parts by mass). The prepared aqueous solution was divided equally into three and one part thereof was added to the above-mentioned powder dispersion. Successively, phytosteryl/octyldodecyl lauroyl glutamate ("Eldew PS-203", manufactured by Ajinomoto Co., Inc.) (0.3 parts by mass) was added, and the mixture was stirred well, alkali aqueous solution of N^{ε}-octanoyl-L-lysine divided equally into four earlier was added and this operation was repeated one more time. The pH of the powder dispersion then was 8.5. The pH of the dispersion was confirm by a pH meter, neutralized with 1 mol/L aqueous sodium hydroxide solution (until pH of about 7.0), stirred for 15 min, filtered and washed with water. Successively, the dispersion was dried for 24 hr by a fan drying machine set to 80°C to give plate barium sulfate (40.7 g) modified with phytosteryl/octyldodecyl lauroyl glutamate and N^{ε}-octanoyl-L-lysine which was used as Example 88.

### [Example 89] N^{ε}-octanoyl-L-lysine modified with fluorine compound

N^{ε}-octanoyl-L-lysine (50 g) was placed in a round-bottle flask (or kneader), an almost 1:1 mixture (2.42 g) of heptadecafluorodecyl phosphoric acid (C₈F₁₇CH₂CH₂P(O) (OH)₂) and diheptadecafluorodecyl phosphoric acid ((C₈F₁₇CH₂CH₂)₂P (O) OH) after dissolution in isopropyl alcohol (500 g) by heating at 50°C was added thereto and they were mixed at 60°C for 4 hr. Thereafter, isopropyl alcohol was evaporated under reduced pressure at 40°C - 50°C and the residue was dried to give a water repellent and oil repellent powder (N^{ε}-octanoyl-L-lysine modified with fluorine compound) (51 g) which was used as Example 89.

### [Example 90] N^{ε}-octanoyl-L-lysine modified with perfluoroalkyl phosphate

N^{ε}-octanoyl-L-lysine (50 g) was placed in a round-bottle flask (or kneader), 15% aqueous solution (6.7 g) of diethanolamine salt of perfluoroalkyl phosphate ("AG-530", manufactured by ASAHI GLASS CO., LTD.) after dissolution in water (500 g) was added thereto and they were mixed at 60°C for 4 hr. Then, hydrochloric acid was added and the mixture was filtered and washed several times with water. This was dried to give N^{ε}-octanoyl-L-lysine (51 g) modified with perfluoroalkyl phosphate which was used as Example 90.

### [Example 91] sericite modified with N^{ε}-octanoyl-L-lysine and diheptadecafluorodecyl phosphoric acid

Sericite (100 g) and N^{ε}-octanoyl-L-lysine (5.0 g) were placed in a Henschel mixer and mixed for 10 min to perform a modification treatment of the above-mentioned sericite, whereby N^{ε}-octanoyl-L-lysine-treated sericite (105 g) was obtained. Then, the aforementioned treated sericite (105 g) was placed in a round-bottle flask, a solution of a triisopropoxytitanium salt of diheptadecafluorodecyl phosphoric acid [[(C₈F₁₇C₂H₄O)₂P(=O)-O-]Ti[(OCH(CH₃)₂]₃] (5.0 g) dissolved in isopropyl alcohol (500 g) by heating was added and the mixture was mixed at 60°C for 4 hr. Thereafter, isopropyl alcohol was evaporated under reduced pressure, and the residue was dried to give sericite (110 g) modified with N^{ε}-octanoyl-L-lysine and diheptadecafluorodecyl phosphoric acid which was used as Example 91.

### [Example 92] sericite modified with N^{ε}-octanoyl-L-lysine and heptadecafluorodecyltriethoxysilane

Sodium hydroxide (3.0 g) was dissolved in 30% ethanol aqueous solution (500 mL), N^{ε}-octanoyl-L-lysine (5.0 g) was added and dissolved at 60°C, and sericite (100 g) was further added. The aforementioned sericite dispersion under mixing was neutralized with 1N hydrochloric acid (75 mL) to allow for precipitation of N^{ε}-octanoyl-L-lysine on the powder. The dispersion was stirred for 30 min, filtered, washed with water to remove water-soluble salt and dried to give N^{ε}-octanoyl-L-lysine-treated sericite (105 g). Next, the aforementioned N^{ε}-octanoyl-L-lysine-treated sericite (105 g) was placed in a round-bottle flask, heptadecafluorodecyltriethoxysilane [(C₈F₁₇C₂H₄) Si (OC₂H₅)₃] (5.0 g) and ethanol (500 g) were added thereto and the mixture was stirred at 60°C for 1 hr. Thereafter, isopropyl alcohol was evaporated under reduced pressure, and the residue was dried to give sericite (110 g) modified with N^{ε}-octanoyl-L-lysine and heptadecafluorodecyltriethoxysilane which was used as Example 92.

### [Example 93] mica modified with N^{ε}-octanoyl-L-lysine and diheptadecafluorodecyl phosphoric acid

N^{ε}-octanoyl-L-lysine (5.0 g) was added to ethanol (2500 g), calcium chloride (2.5 g) was further added and the mixture was stirred to dissolve N^{ε}-octanoyl-L-lysine as a calcium salt. Mica (100 g) was added and the mixture was stirred at room temperature for 2 hr. The mixture was filtered, washed with water to remove calcium chloride and dried to give mica (102 g) treated for modification with N^{ε}-octanoyl-L-lysine.Then, the aforementioned N^{ε}-octanoyl-L-lysine-treated mica (102 g) was placed in a round-bottle flask, a triisopropoxytitanium salt of diheptadecafluorodecyl phosphoric acid [[(C₈F₁₇C₂H₄O)₂P(=O)-O-]Ti[(OCH(CH₃)₂]₃] (5.0 g) was added together with isopropyl alcohol (500 g) and the mixture was stirred at 60°C for 1 hr. Thereafter, isopropyl alcohol was evaporated under reduced pressure, and the residue was dried to give mica (105 g) modified with N^{ε}-octanoyl-L-lysine and diheptadecafluorodecyl phosphoric acid which was used as Example 93.

The composite powders of Examples 1 - 93 are all superior in dispersibility and caking property when blended with makeup cosmetics, achieve superior texture (e.g., smoothness, moist feeling etc.) when applied to the skin, and are superior in water-repellency, adhesiveness and transparency.

Formulation Examples of the makeup cosmetic of the present invention are shown in the following.

### [Formulation Examples 1 - 11] sunscreening cream

Formulation Examples of sunscreening cream are shown in Table 1. The numerical values in Table 1 show the contents (%) of respective components.

**Table 1]**

| | component | Form. Ex. 1 | Form. Ex. 2 | Form. Ex. 3 | Form. Ex. 4 | Form. Ex. 5 | Form. Ex. 6 | Form. Ex. 7 | Form. Ex. 8 | Form. Ex. 9 | Form. Ex. 10 | Form. Ex. 11 |
|---|---|---|---|---|---|---|---|---|---|---|---|---|
| (1) | methylpolysiloxane | 10.0 | 10.0 | 10.0 | 10.0 | 10.0 | 10.0 | 10.0 | 10.0 | 10.0 | 10.0 | 10.0 |
| (2) | octyldodecyl/phytosteryl/ behenyl lauroyl glutamate | 1.0 | 1.0 | 1.0 | 1.0 | 1.0 | 1.0 | 1.0 | 1.0 | 1.0 | 1.0 | 1.0 |
| (3) | d-α-tocopherol acetate | 0.1 | 0.1 | 0.1 | 0.1 | 0.1 | 0.1 | 0.1 | 0.1 | 0.1 | 0.1 | 0.1 |
| (4) | polyoxyalkylene denatured dimethylpolysiloxane, dimethylpolysiloxane | 2.0 | 2.0 | 2.0 | 2.0 | 2.0 | 2.0 | 2.0 | 2.0 | 2.0 | 2.0 | 2.0 |
| (5) | decamethylcyclopentasiloxane | 15.0 | 15.0 | 15.0 | 15.0 | 15.0 | 15.0 | 15.0 | 15.0 | 15.0 | 15.0 | 15.0 |
| (6) | titanium oxide fine particles | 5.0 | 5.0 | 5.0 | 5.0 | 5.0 | 5.0 | 5.0 | 5.0 | 5.0 | 5.0 | 5.0 |
| | zinc oxide | | | | | 10.0 | 10.0 | 10.0 | 10.0 | 10.0 | 10.0 | 10.0 |
| | composite powder of Example 10 | 13.0 | | | | | | | | | | |
| | composite powder of Example 11 | | 13.0 | | | | | | | | | |
| | composite powder of Example 12 | | | 13.0 | | | | | | | | |
| | composite powder of Example 13 | | | | 13.0 | | | | | | | |
| | composite powder of Example 14 | | | | | 3.0 | | | | | | |
| | composite powder of Example 15 | | | | | | 3.0 | | | | | |
| | composite powder of Example 51 | | | | | | | 3.0 | | | | |
| | composite powder of Example 52 | | | | | | | | 3.0 | | | |
| | composite powder of Example 88 | | | | | | | | | 3.0 | | |
| | composite powder of Example 89 | | | | | | | | | | 3.0 | |
| | composite powder of Example 90 | | | | | | | | | | | 3.0 |
| (7) | anhydrous magnesium sulfate | 0.5 | 0.5 | 0.5 | 0.5 | 0.5 | 0.5 | 0.5 | 0.5 | 0.5 | 0.5 | 0.5 |
| (8) | sodium pyrrolidone carboxylate (50%) | 0.5 | 0.5 | 0.5 | 0.5 | 0.5 | 0.5 | 0.5 | 0.5 | 0.5 | 0.5 | 0.5 |
| (9) | glycerol | 5.0 | 5.0 | 5.0 | 5.0 | 5.0 | 5.0 | 5.0 | 5.0 | 5.0 | 5.0 | 5.0 |
| (10) | hydroxyethylcellulose | 0.1 | 0.1 | 0.1 | 0.1 | 0.1 | 0.1 | 0.1 | 0.1 | 0.1 | 0.1 | 0.1 |
| (11) | 1,3-butyleneglycol | 5.0 | 5.0 | 5.0 | 5.0 | 5.0 | 5.0 | 5.0 | 5.0 | 5.0 | 5.0 | 5.0 |
| (12) | phenoxyethanol | 0.3 | 0.3 | 0.3 | 0.3 | 0.3 | 0.3 | 0.3 | 0.3 | 0.3 | 0.3 | 0.3 |
| (13) | water | 42.5 | 42.5 | 42.5 | 42.5 | 42.5 | 42.5 | 42.5 | 42.5 | 42.5 | 42.5 | 42.5 |

Sunscreening cream can be prepared according to the following procedure.
(i) Components (1) - (4) in Table 1 are mixed by stirring at 70°C.
(ii) After cooling the mixture prepared in (i), component (5) is mixed by stirring at room temperature.
(iii) Component (6) is uniformly dispersed (disper, 3000 rpm, 10 min) at room temperature.
(iv) Components (7) - (13) are dissolved by mixing by stirring at room temperature.
(v) While rotating a homomixer at room temperature, the solution prepared in (iv) is gradually added to the mixture of components (1) - (6) prepared in (iii) and the mixture is emulsified.

### [Formulation Examples 12 - 25] two-way powder foundation

Formulation Examples of two-way powder foundation are shown in Table 2. The numerical values in Table 2 show the contents (%) of respective components.

**[Table 2]**

| | component | Form. Ex. 12 | Form. Ex. 13 | Form. Ex. 14 | Form. Ex. 15 | Form. Ex. 16 | Form. Ex. 17 | Form. Ex. 18 | Form. Ex. 19 | Form. Ex. 20 | Form. Ex. 21 | Form. Ex. 22 | Form. Ex. 23 | Form. Ex. 24 | Form. Ex. 25 |
|---|---|---|---|---|---|---|---|---|---|---|---|---|---|---|---|
| (1) | red iron oxide | 1.5 | 1.5 | 1.5 | 1.5 | 1.5 | 1.5 | 1.5 | 1.5 | 1.5 | 1.5 | 1.5 | 1.5 | 1.5 | 1.5 |
| (2) | yellow iron oxide | 2.8 | 2.8 | 2.8 | 2.8 | 2.8 | 2.8 | 2.8 | 2.8 | 2.8 | 2.8 | 2.8 | 2.8 | 2.8 | 2.8 |
| (3) | black iron oxide | 0.1 | 0.1 | 0.1 | 0.1 | 0.1 | 0.1 | 0.1 | 0.1 | 0.1 | 0.1 | 0.1 | 0.1 | 0.1 | 0.1 |
| (4) | titanium oxide | 8.0 | 8.0 | 8.0 | 8.0 | 8.0 | 8.0 | 8.0 | 8.0 | 8.0 | 8.0 | 8.0 | 8.0 | 8.0 | 8.0 |
| (5) | sericite | 26.0 | 26.0 | 26.0 | 26.0 | 26.0 | 26.0 | 26.0 | 26.0 | 26.0 | 26.0 | 26.0 | 26.0 | 26.0 | 26.0 |
| (6) | talc | rest | rest | rest | rest | rest | rest | rest | rest | rest | rest | rest | rest | rest | rest |
| (7) | composite powder of Ex. 1 | 6.0 | | | | | | | | | | | | | |
| | composite powder of Ex. 2 | | 6.0 | | | | | | | | | | | | |
| | composite powder of Ex. 3 | | | 6.0 | | | | | | | | | | | |
| | composite powder of Ex. 20 | | | | 6.0 | | | | | | | | | | |
| | composite powder of Ex. 26 | | | | | 6.0 | | | | | | | | | |
| | composite powder of Ex. 29 | | | | | | 6.0 | | | | | | | | |
| | composite powder of Ex. 36 | | | | | | | 6.0 | | | | | | | |
| | composite powder of Ex. 43 | | | | | | | | 6.0 | | | | | | |
| | composite powder of Ex. 48 | | | | | | | | | 6.0 | | | | | |
| | composite powder of Ex. 57 | | | | | | | | | | 6.0 | | | | |
| | composite powder of Ex. 58 | | | | | | | | | | | 6.0 | | | |
| | composite powder of Ex. 61 | | | | | | | | | | | | 6.0 | | |
| | composite powder of Ex. 80 | | | | | | | | | | | | | 6.0 | |
| | composite powder of Ex. 93 | | | | | | | | | | | | | | 6.0 |
| (8) | octyldodecyl myristate | 5.7 | 5.7 | 5.7 | 5.7 | 5.7 | 5.7 | 5.7 | 5.7 | 5.7 | 5.7 | 5.7 | 5.7 | 5.7 | 5.7 |
| (9) | squalane | 2.5 | 2.5 | 2.5 | 2.5 | 2.5 | 2.5 | 2.5 | 2.5 | 2.5 | 2.5 | 2.5 | 2.5 | 2.5 | 2.5 |
| (10) | methylphenyl-polysiloxane | 5.3 | 5.3 | 5.3 | 5.3 | 5.3 | 5.3 | 5.3 | 5.3 | 5.3 | 5.3 | 5.3 | 5.3 | 5.3 | 5.3 |
| (11) | preservative | q.s. | q.s. | q.s. | q.s. | q.s. | q.s. | q.s. | q.s. | q.s. | q.s. | q.s. | q.s. | q.s. | q.s. |
| (12) | flavor | q.s. | q.s. | q.s. | q.s. | q.s. | q.s. | q.s. | q.s. | q.s. | q.s. | q.s. | q.s. | q.s. | q.s. |

Two-way powder foundation can be prepared according to the following procedure.
(i) Components (1) - (7) in Table 2 are mixed and pulverized by passing through a grinding machine.
(ii) The pulverized product is transferred to a high-speed blender. Components (8) - (12) are mixed and heated to give a uniform mixture, and the mixture is added to the pulverized product and they are further mixed to give a uniform mixture.
(iii) The mixture is pulverized by a grinding machine, passed through a sieve to adjust the particle size, and compression molded.

### [Formulation Examples 26 - 34] emulsion foundation

Formulation Examples of emulsion foundation are shown in Table 3. The numerical values in Table 3 show the contents (%) of respective components.

**[Table 3]**

| | component | Form. Ex. 26 | Form. Ex. 27 | Form. Ex. 28 | Form. Ex. 29 | Form. Ex. 30 | Form. Ex. 31 | Form. Ex. 32 | Form. Ex. 33 | Form. Ex. 34 |
|---|---|---|---|---|---|---|---|---|---|---|
| (1) | decamethylcyclopentasiloxane | 22 | 22 | 22 | 22 | 22 | 22 | 22 | 22 | 22 |
| (2) | petrolatum | 2 | 2 | 2 | 2 | 2 | 2 | 2 | 2 | 2 |
| (3) | methylphenylpolysiloxane | 6 | 6 | 6 | 6 | 6 | 6 | 6 | 6 | 6 |
| (4) | squalane | 5 | 5 | 5 | 5 | 5 | 5 | 5 | 5 | 5 |
| (5) | isooctyl isononanoate | 6 | 6 | 6 | 6 | 6 | 6 | 6 | 6 | 6 |
| (6) | dimethylpolysiloxane polyoxyalkylene polymer (HLB=3.5) | 3.5 | 3.5 | 3.5 | 3.5 | 3.5 | 3.5 | 3.5 | 3.5 | 3.5 |
| (7) | composite powder of Example 16 | 1.6 | | | | | | | | |
| | composite powder of Example 31 | | 1.6 | | | | | | | |
| | composite powder of Example 38 | | | 1.6 | | | | | | |
| | composite powder of Example 45 | | | | 1.6 | | | | | |
| | composite powder of Example 53 | | | | | 1.6 | 1.6 | | | |
| | composite powder of Example 65 | | | | | | | 1.6 | | |
| | composite powder of Example 70 | | | | | | | | 1.6 | |
| | composite powder of Example 82 | | | | | | | | | 1.6 |
| (8) | composite powder of Example 17 | 2.5 | | | | | | | | |
| | composite powder of Example 32 | | 2.5 | | | | | | | |
| | composite powder of Example 39 | | | 2.5 | | | | | | |
| | composite powder of Example 46 | | | | 2.5 | | | | | |
| | composite powder of Example 54 | | | | | 2.5 | 2.5 | | | |
| | composite powder of Example 66 | | | | | | | 2.5 | | |
| | composite powder of Example 71 | | | | | | | | 2.5 | |
| | composite powder of Example 83 | | | | | | | | | 2.5 |
| (9) | composite powder of Example 18 | 0.1 | | | | | | | | |
| | composite powder of Example 33 | | 0.1 | | | | | | | |
| | composite powder of Example 40 | | | 0.1 | | | | | | |
| | composite powder of Example 47 | | | | 0.1 | | | | | |
| | composite powder of Example 55 | | | | | 0.1 | | | | |
| | composite powder of Example 60 | | | | | | 0.1 | | | |
| | composite powder of Example 67 | | | | | | | 0.1 | | |
| | composite powder of Example 72 | | | | | | | | 0.1 | |
| | composite powder of Example 84 | | | | | | | | | 0.1 |
| (10) | titanium oxide | 8.5 | 8.5 | 8.5 | 8.5 | 8.5 | 8.5 | 8.5 | 8.5 | 8.5 |
| (11) | talc | 2.5 | 2.5 | 2.5 | 2.5 | 2.5 | 2.5 | 2.5 | 2.5 | 2.5 |
| (12) | ethanol | 5 | 5 | 5 | 5 | 5 | 5 | 5 | 5 | 5 |
| (13) | 1,3-butyleneglycol | 5 | 5 | 5 | 5 | 5 | 5 | 5 | 5 | 5 |
| (14) | sodium chloride | 2 | 2 | 2 | 2 | 2 | 2 | 2 | 2 | 2 |
| (15) | purified water | rest | rest | rest | rest | rest | rest | rest | rest | rest |
| (16) | preservative | q.s. | q.s. | q.s. | q.s. | q.s. | q.s. | q.s. | q.s. | q.s. |
| (17) | flavor | q.s. | q.s. | q.s. | q.s. | q.s. | q.s. | q.s. | q.s. | q.s. |

Emulsion foundation can be prepared according to the following procedure.
(i) Components (7) - (11) in Table 3 are mixed and pulverized in advance.
(ii) Components (1) - (6) are mixed at 70°C, to the uniformly dissolved oil phase is added the mixture of components (7) - (11) prepared in (i), and the mixture is uniformly dispersed by a homodisper.
(iii) Components (12) - (16) are mixed at 70°C, the uniformly dissolved aqueous phase is gradually added to the aforementioned oil phase, and the mixture is uniformly dispersed by a homomixer and cooled.
(iv) Component (17) is added and emulsion particles are settled.

### [Formulation Examples 35 - 57] solid face powder

Formulation Examples of solid face powder are shown in Tables 4 -1 and 4-2. The numerical values in Tables 4 -1 and 4-2 show the contents (%) of respective components.

**[Table 4-1]**

| | component | Form. Ex. 35 | Form. Ex. 36 | Form. Ex. 37 | Form. Ex. 38 | Form. Ex. 39 | Form. Ex. 40 | Form. Ex. 41 | Form. Ex. 42 | Form. Ex. 43 | Form. Ex. 44 | Form. Ex. 45 | Form. Ex. 46 | Form. Ex. 47 |
|---|---|---|---|---|---|---|---|---|---|---|---|---|---|---|
| (1) | mica | 25.0 | 25.0 | 25.0 | 25.0 | 25.0 | 25.0 | 25.0 | 25.0 | 25.0 | 25.0 | 25.0 | 25.0 | 25.0 |
| (2) | talc | rest | rest | rest | rest | rest | rest | rest | rest | rest | rest | rest | rest | rest |
| (3) | sericite | 10.0 | 10.0 | 10.0 | 10.0 | 10.0 | 10.0 | 10.0 | 10.0 | 10.0 | 10.0 | 10.0 | 10.0 | 10.0 |
| | composite powder of Ex. 4 | 1.5 | | | | | | | | | | | | |
| | composite powder of Ex. 5 | | 1.5 | | | | | | | | | | | |
| | composite powder of Ex. 6 | | | 1.5 | | | | | | | | | | |
| | composite powder of Ex. 21 | | | | 1.5 | | | | | | | | | |
| | composite powder of Ex. 23 | | | | | 1.5 | | | | | | | | |
| | composite powder of Ex. 27 | | | | | | 1.5 | | | | | | | |
| | composite powder of Ex. 34 | | | | | | | 1.5 | | | | | | |
| | composite powder of Ex. 41 | | | | | | | | 1.5 | | | | | |
| | composite powder of Ex. 49 | | | | | | | | | 1.5 | | | | |
| | composite powder of Ex. 59 | | | | | | | | | | 1.5 | | | |
| | composite powder of Ex. 62 | | | | | | | | | | | 1.5 | | |
| (4) | composite powder of Ex. 63 | | | | | | | | | | | | 1.5 | |
| | composite powder of Ex. 64 | | | | | | | | | | | | | 1.5 |
| | composite powder of Ex. 68 | | | | | | | | | | | | | |
| | composite powder of Ex. 69 | | | | | | | | | | | | | |
| | composite powder of Ex. 73 | | | | | | | | | | | | | |
| | composite powder of Ex. 74 | | | | | | | | | | | | | |
| | composite powder of Ex. 78 | | | | | | | | | | | | | |
| | composite powder of Ex. 79 | | | | | | | | | | | | | |
| | composite powder of Ex. 81 | | | | | | | | | | | | | |
| | composite powder of Ex. 85 | | | | | | | | | | | | | |
| | composite powder of Ex. 86 | | | | | | | | | | | | | |
| | composite powder of Ex. 87 | | | | | | | | | | | | | |
| (5) | flavor | q.s. | q.s. | q.s. | q.s. | q.s. | q.s. | q.s. | q.s. | q.s. | q.s. | q.s. | q.s. | q.s. |

**[Table 4-2]**

| | component | Form. Ex. 48 | Form. Ex. 49 | Form. Ex. 50 | Form. Ex. 51 | Form. Ex. 52 | Form. Ex. 53 | Form. Ex. 54 | Form. Ex. 55 | Form. Ex. 56 | Form. Ex. 57 |
|---|---|---|---|---|---|---|---|---|---|---|---|
| (1) | mica | 25.0 | 25.0 | 25.0 | 25.0 | 25.0 | 25.0 | 25.0 | 25.0 | 25.0 | 25.0 |
| (2) | talc | rest | rest | rest | rest | rest | rest | rest | rest | rest | rest |
| (3) | sericite | 10.0 | 10.0 | 10.0 | 10.0 | 10.0 | 10.0 | 10.0 | 10.0 | 10.0 | 10.0 |
| | composite powder of Ex. 4 | | | | | | | | | | |
| | composite powder of Ex. 5 | | | | | | | | | | |
| | composite powder of Ex. 6 | | | | | | | | | | |
| | composite powder of Ex. 21 | | | | | | | | | | |
| | composite powder of Ex. 23 | | | | | | | | | | |
| | composite powder of Ex. 27 | | | | | | | | | | |
| | composite powder of Ex. 34 | | | | | | | | | | |
| | composite powder of Ex. 41 | | | | | | | | | | |
| | composite powder of Ex. 49 | | | | | | | | | | |
| | composite powder of Ex. 59 | | | | | | | | | | |
| | composite powder of Ex. 62 | | | | | | | | | | |
| (4) | composite powder of Ex. 63 | | | | | | | | | | |
| | composite powder of Ex. 64 | | | | | | | | | | |
| | composite powder of Ex. 68 | 1.5 | | | | | | | | | |
| | composite powder of Ex. 69 | | 1.5 | | | | | | | | |
| | composite powder of Ex. 73 | | | 1.5 | | | | | | | |
| | composite powder of Ex. 74 | | | | 1.5 | | | | | | |
| | composite powder of Ex. 78 | | | | | 1.5 | | | | | |
| | composite powder of Ex. 79 | | | | | | 1.5 | | | | |
| | composite powder of Ex. 81 | | | | | | | 1.5 | | | |
| | composite powder of Ex. 85 | | | | | | | | 1.5 | | |
| | composite powder of Ex. 86 | | | | | | | | | 1.5 | |
| | composite powder of Ex. 87 | | | | | | | | | | 1.5 |
| (5) | flavor | q.s. | q.s. | q.s. | q.s. | q.s. | q.s. | q.s. | q.s. | q.s. | q.s. |

Solid face powder can be prepared according to the following procedure.
(i) Components (1) - (5) in Tables 4 -1 and 4-2 are uniformly mixed.
(ii) Ethanol is added and the mixture is uniformly mixed.
(iii) The mixture is filled in an inner tray and suction-compression molded.
(iv) The molded product is dried at 40°C for 24 hr.

### [Formulation Examples 58 - 66] powder eyeshadow

Formulation Examples of powder eyeshadow are shown in Table 5. The numerical values in Table 5 show the contents (%) of respective components.

**[Table 5]**

| | component | Form. Ex. 58 | Form. Ex. 59 | Form. Ex. 60 | Form. Ex. 61 | Form. Ex. 62 | Form. Ex. 63 | Form. Ex. 64 | Form. Ex. 65 | Form. Ex. 66 |
|---|---|---|---|---|---|---|---|---|---|---|
| (1) | composite powder of Example 19 | 25 | | | | | | | | |
| | composite powder of Example 22 | | 25 | | | | | | | |
| | composite powder of Example 24 | | | 25 | | | | | | |
| | composite powder of Example 28 | | | | 25 | | | | | |
| | composite powder of Example 35 | | | | | 25 | | | | |
| | composite powder of Example 42 | | | | | | 25 | | | |
| | composite powder of Example 56 | | | | | | | 25 | | |
| | composite powder of Example 91 | | | | | | | | 25 | |
| | composite powder of Example 92 | | | | | | | | | 25 |
| (2) | mica | 10.0 | 10.0 | 10.0 | 10.0 | 10.0 | 10.0 | 10.0 | 10.0 | 10.0 |
| (3) | mica titanium | 6.5 | 6.5 | 6.5 | 6.5 | 6.5 | 6.5 | 6.5 | 6.5 | 6.5 |
| (4) | color pigment | q.s. | q.s. | q.s. | q.s. | q.s. | q.s. | q.s. | q.s. | q.s. |
| (5) | squalane | 8.7 | 8.7 | 8.7 | 8.7 | 8.7 | 8.7 | 8.7 | 8.7 | 8.7 |
| (6) | dimethylpolysiloxane | 2.5 | 2.5 | 2.5 | 2.5 | 2.5 | 2.5 | 2.5 | 2.5 | 2.5 |
| (7) | polyethylene glycol/octyldodecyl myristate | 8.0 | 8.0 | 8.0 | 8.0 | 8.0 | 8.0 | 8.0 | 8.0 | 8.0 |
| (8) | ceresin wax | 3.5 | 3.5 | 3.5 | 3.5 | 3.5 | 3.5 | 3.5 | 3.5 | 3.5 |
| (9) | sorbitan tristearate | 1.0 | 1.0 | 1.0 | 1.0 | 1.0 | 1.0 | 1.0 | 1.0 | 1.0 |
| (10) | flavor | q.s. | q.s. | q.s. | q.s. | q.s. | q.s. | q.s. | q.s. | q.s. |

Powder eyeshadow can be prepared according to the following procedure.
(i) Among the components in Table 5, components (1), (2), (4) are mixed in a Henschel mixer and pulverized by an atomizer.
(ii) Separately, components (5) - (10) are mixed and uniformly dissolved.
(iii) The mixed ground product of (i) is mixed with component (3), the solution prepared in (ii) is added and they are uniformly mixed.
(iii) The mixture is pulverized by an atomizer, passed through a sieve, filled in an inner tray and compression molded.

### [Formulation Examples 67 - 74] oil foundation

Formulation Examples of oil foundation are shown in Table 6. The numerical values in Table 6 show the contents (%) of respective components.

**[Table 6]**

| | component | Form. Ex. 67 | Form. Ex. 68 | Form. Ex. 69 | Form. Ex. 70 | Form. Ex. 71 | Form. Ex. 72 | Form. Ex. 73 | Form. Ex. 74 |
|---|---|---|---|---|---|---|---|---|---|
| (1) | isopropyl palmitate | 20.0 | 20.0 | 20.0 | 20.0 | 20.0 | 20.0 | 20.0 | 20.0 |
| (2) | cetanol | 7.0 | 7.0 | 7.0 | 7.0 | 7.0 | 7.0 | 7.0 | 7.0 |
| (3) | squalane | 15.5 | 15.5 | 15.5 | 15.5 | 15.5 | 15.5 | 15.5 | 15.5 |
| (4) | polyglyceryl triisostearate | 5.0 | 5.0 | 5.0 | 5.0 | 5.0 | 5.0 | 5.0 | 5.0 |
| (5) | volatility liquid paraffin | 10.0 | 10.0 | 10.0 | 10.0 | 10.0 | 10.0 | 10.0 | 10.0 |
| (6) | ceresin wax | 3.3 | 3.3 | 3.3 | 3.3 | 3.3 | 3.3 | 3.3 | 3.3 |
| (7) | candelilla wax | 4.5 | 4.5 | 4.5 | 4.5 | 4.5 | 4.5 | 4.5 | 4.5 |
| (8) | color pigment | q.s. | q.s. | q.s. | q.s. | q.s. | q.s. | q.s. | q.s. |
| (9) | titanium oxide | 15.5 | 15.5 | 15.5 | 15.5 | 15.5 | 15.5 | 15.5 | 15.5 |
| (10) | composite powder of Example 7 | 8 | | | | | | | |
| | composite powder of Example 8 | | 8 | | | | | | |
| | composite powder of Example 9 | | | 8 | | | | | |
| | composite powder of Example 25 | | | | 8 | | | | |
| | composite powder of Example 30 | | | | | 8 | | | |
| | composite powder of Example 37 | | | | | | 8 | | |
| | composite powder of Example 44 | | | | | | | 8 | |
| | composite powder of Example 50 | | | | | | | | 8 |
| (11) | preservative | q.s. | q.s. | q.s. | q.s. | q.s. | q.s. | q.s. | q.s. |
| (12) | flavor | q.s. | q.s. | q.s. | q.s. | q.s. | q.s. | q.s. | q.s. |

Oil foundation can be prepared according to the following procedure.
(i) Components (8) - (10) in Table 6 are mixed and pulverized in advance.
(ii) Components (1) - (7), (11) are mixed at 85°C, to the dissolved oil phase are added components (8) - (10) mixed and pulverized in (i), and the mixture is uniformly dispersed by a homodisper.
(iii) Component (12) is added and mixed, and the mixture is filled in a metal tray and cooled.

The makeup cosmetics of Formulation Examples 1 - 74 all achieve superior texture (e.g., smoothness, moist feeling) when applied to the skin, and are superior in water-repellency, adhesiveness and transparency.

### [Industrial Applicability]

As described in detail above, according to the present invention, a powder modifying agent that can impart a powder for cosmetics with the properties appropriate as a powder for makeup cosmetics can be provided.

That is, the powder modifying agent of the present invention can impart a powder for cosmetics with superior dispersibility and superior caking property when blended with makeup cosmetics, superior texture (e.g., smoothness, moist feeling etc.) when applied to the skin, and water-repellency, adhesiveness and transparency.

A composite powder prepared using the powder modifying agent of the present invention can exhibit the above-mentioned properties in good balance when blended with makeup cosmetics. Thus, makeup cosmetics containing the composite powder are superior in the texture when applied to the skin and superior in water-repellency, adhesiveness and transparency.

This application is based on a patent application No. 2017-114444 filed in Japan, the contents of which are incorporated in full herein.

## Claims

1. A powder modifying agent comprising N^{ε}-octanoyl-L-lysine.

2. A composite powder comprising a powder core and N^{ε}-octanoyl-L-lysine.

3. The composite powder according to claim 2 comprising 0.1 mass % - 50 mass % of the N^{ε}-octanoyl-L-lysine.

4. The composite powder according to claim 2 or 3 having an average particle size of 0.001 µm - 400 µm and a specific surface area of 1 m²/g - 700 m²/g.

5. The composite powder according to any one of claims 2 to 4 wherein the powder core is in the form of a plate.

6. The composite powder according to any one of claims 2 to 5 wherein the powder core is one or more kinds selected from inorganic powders.

7. The composite powder according to any one of claims 2 to 6 wherein the powder core is one or more kinds selected from titanium oxide, zinc oxide, talc, mica, fluorphlogopite and silica.

8. The composite powder according to any one of claims 2 to 7 further comprising N^{ε}-lauroyl-L-lysine.

9. A method for producing the composite powder according to any one of claims 2 to 7 comprising modifying the powder core by a surface treatment with N^{ε}-octanoyl-L-lysine in water.

10. The production method according to claim 8 wherein the modification of the powder core is performed by a stepwise surface treatment with N^{ε}-octanoyl-L-lysine and N^{ε}-lauroyl-L-lysine in water.

11. The production method according to claim 8 wherein the modification of the powder core is performed by a simultaneously surface treatment with N^{ε}-octanoyl-L-lysine and N^{ε}-lauroyl-L-lysine in water.

12. The composite powder according to any one of claims 2 to 8 wherein the composite powder is used as a pigment for cosmetics.

13. The makeup cosmetic comprising the composite powder according to any one of claims 2 to 8.
